# EUROPEAN PATENT APPLICATION

(11) **EP 1 493 455 A2**
(43) Date of publication of application: **05.01.2005**
(21) Application number: 04253363.8
(22) Date of filing: 04.06.2004
(51) Int. Cl.: A61L 27/16

(54) **Crosslinked polymeric composite for orthopaedic implants**

(30) Priority: 30.06.2003 US 610161
(71) Applicant: Depuy Products, Inc., Warsaw, Indiana 46581 (US)
(72) Inventor: Smith, Todd S., Ft Wayne IN 46804 (US); King, Richard, Warsaw IN 46580 (US); McNulty, Donald E., Warsaw IN 46582 (US)
(74) Representative: Belcher, Simon James

(57) **Abstract**

An implantable orthopaedic device has a bearing component made from a crosslinked polymeric material which is substantially free of free radicals and a backing component having a plurality of pores defined therein. The backing component (i) is made from a material that has a greater modulus of elasticity than the bearing component and (ii) is attached to the polymeric bearing component by a mass of the crosslinked polymeric material interdigitated within the pores of the backing component. The device can be made by a method which includes advancing a mass of the polymeric material of the consolidated bearing component into the pores of the backing component to cause the backing component to be mechanically attached to the bearing component.

## Description

The present disclosure generally relates to orthopaedic devices for implantation in the body of an animal and associated methods for making the same. The present disclosure particularly relates to orthopaedic devices for implantation in the body of an animal that include a crosslinked polymeric/metallic or ceramic composite and associated methods for making the same.

The articulation of metallic components against one another in an implantable orthopaedic device can cause the device to have a high wear rate. The substitution of a polymeric component for one of the metallic components can dramatically reduce the wear rate of the orthopaedic device. Accordingly, orthopaedic devices that include modular composite arrangements have evolved. For example, these modular composite arrangements can include (i) a modular bearing component made of a polymeric material secured or locked to (ii) a modular backing component made of a metallic material. The modular bearing component made of a polymeric material enhances the wear performance of the orthopaedic device while the modular backing component made of a metallic material provides a more uniform stress transfer to the anchoring bone and allows the in growth of bone into the backing component to enhance fixation.

As indicated above, these modular composite arrangements include a modular locking mechanism for securing the polymeric bearing component to the metallic backing component. For example, these locking mechanisms can include the use of a pin or a snap fit locking arrangement to secure polymeric bearing component to the metallic backing component. However, some of the locking mechanisms utilized in these modular composite arrangements allow undesirable relative movement between the polymeric bearing component and the metallic backing component of the orthopaedic device. For example, both micro and macro relative motion at the interface of the polymeric bearing component and the metallic backing component can occur. Accordingly, a modular composite arrangement that has appropriate wear and stress distribution characteristics, in addition to enhanced fixation characteristics between the polymeric bearing component and the backing component is desirable.

An implantable orthopaedic device and a method of preparing an implantable orthopaedic device, such as knee, hip, shoulder, and elbow prostheses, in accordance with the present disclosure comprises one or more of the features or combinations thereof:

An implantable orthopaedic device includes a composite arrangement, such as a modular composite arrangement, having a polymeric component secured to a backing component. The backing component can be made from a rigid porous material that has a greater modulus of elasticity than the bearing component. The backing component can be made from any suitable biocompatible rigid material that can be fabricated with a textured portion, with a porosity layer, a porous coating, or fabricated such that the backing component is completely porous. The backing component can have an open porous structure where the pores of the backing component can form a three dimensional network of continuously substantially connected channels. The pores of the backing component can form a three dimensional network of continuously substantially connected channels that define a bulk volume porosity within a range of from about 25% to about 75%. For example, the pores of the backing component can form a three dimensional network of continuously substantially connected channels that define a bulk volume porosity within a range of from about 30% to about 65%, or about 40% to about 50%. In the alternative, the pores of the backing component can have a closed porous structure where the pores are not substantially interconnected. Materials the backing component can be fabricated from include, for example, porous metal, ceramic, polymeric materials, or a combination of these materials. Examples of metals the backing component can be fabricated from include, but are not limited to, titanium alloys and CoCr alloys. Examples of ceramic materials the backing component can be fabricated from include, but are not limited to, alumina, zirconia, or blends of these ceramic materials. In addition, the backing component can be fabricated from various combinations of the aforementioned materials. For example, backing component can include combinations of metal, ceramic, or polymer materials.

The polymeric bearing component can, for example, be a polymeric bearing component. The bearing component can be made from a polymeric material having enhanced bearing surface properties as compared to the material of the backing component. In particular, bearing component can be made from any medical grade polymeric material which may be implanted into the body of an animal (e.g. the body of a human patient) and be capable of having its viscosity reduced sufficiently with the application of thermal energy such that it will deformation flow into, for example, the pores of the backing component. For example, the polymeric material can be compression moulded into the pores of the backing component. A specific example of such a polymeric material is medical grade polyethylene such as a polyethylene homopolymer, high density polyethylene, high molecular weight polyethylene, high density high molecular weight polyethylene, or any other type of polyethylene utilized in the construction of a prosthetic implant. A more specific example of such a polymer is medical grade ultrahigh molecular weight polyethylene (UHMWPE), such as crosslinked UHMWPE.

The polymeric material from which the bearing component is made can be consolidated into a polymeric work piece prior to being secured to the backing component. In addition, after consolidation, the polymeric work piece can be subjected to a crosslinking process. For example, exposing the consolidated polymeric work piece to radiation such as gamma radiation, electron beam, or X-rays will cause the crosslinking of polymeric material. Such exposure may be in the exemplary range of about 0.5 Mrads to about 150 Mrads, illustratively from about 3 to about 50 Mrads, and illustratively from about 3 to about 15 Mrads. In addition, the polymeric material can be subjected to chemical method of crosslinking, such as one that utilizes peroxides. A specific example of a crosslinked polymeric material that can be utilized in the construction of a device to be implanted in the body of an animal, such as the bearing component described herein, is crosslinked UHMWPE, such as highly crosslinked UHMWPE.

After crosslinking the polymeric material, such as UHMWPE, it may be subjected to a post-irradiation free radical quenching process. For example, the free radical containing polymeric work piece can be placed into a vacuum oven under reduced pressure. To quench substantially all the free radicals present in the polymeric work piece, the temperature of the vacuum oven can then be raised to above the melting point of the polymeric material (e.g. greater than 135°C) until it is completely melted, for example about 24 hours. In any event, the polymeric material subjected to a post-irradiation free radical quenching process will be substantially free of free radicals.

The polymeric bearing component can be attached to the backing component. For example, after consolidation and crosslinking, the polymeric bearing component can be attached to the backing component. In particular, the bearing component can be heated to supply the thermal energy necessary to sufficiently reduce the viscosity of the polymeric material such that it becomes impregnated into the pores of the backing component with the application of pressure (e.g. with a compression moulding apparatus). One way of accomplishing this is to elevate the temperature of the backing component to a level at which sufficient thermal energy is transferred to the polymeric bearing component so as to locally reduce the viscosity of the polymeric material. The lowering of the viscosity of the polymeric material reduces the mechanical properties of the material such that with the application of sufficient force the polymeric material interdigitates with the pores of the backing component. The interdigitated polymeric material within the pores of the rigid backing component results in a mechanical bond of sufficient strength to hold the polymeric bearing component secure to the rigid backing component. Accordingly, a backing component having any degree of porosity is contemplated as long as the strength of the interface formed by the interdigitated polymeric material within the pores of the rigid backing component results in a mechanical bond of sufficient strength such that the modular composite arrangement can be implanted in the body of an animal, such as a human being.

Accordingly, the present invention provides a method of securing a consolidated, crosslinked polymeric bearing component to a rigid, porous, backing component. Briefly stated, a consolidated, crosslinked, polymeric bearing component produced by moulding or machining is attached to a porous backing component by forcing the polymeric material of the consolidated bearing component into the pores of the porous backing component. For example, the polymeric bearing component can be heated to supply the thermal energy necessary to sufficiently reduce the viscosity of the polymeric material such that it becomes impregnated into the pores of the backing component with the application of pressure. In particular, the reduction of the viscosity of the polymeric material allows the material to deformation flow. Accordingly, with the application of sufficient pressure, the polymeric material deformation flows into the cavities and interstices of the backing component. Upon returning to ambient temperature, the viscosity of the material reverts back to the original value. At the original viscosity level, the resistance to deformation flow returns to, or very near, the original resistance level. As such, the interdigitated polymeric material within the pores of the rigid backing component results in a mechanical bond of sufficient strength to hold the polymeric bearing component secure to the rigid backing component. In particular, the mechanical strength of the bond between the polymeric bearing component and the porous metallic or ceramic backing component at ambient or body temperature is such that both micro and macro relative motion at the interface between these two components is substantially inhibited. Accordingly, as discussed in greater detail below, the crosslinked polymeric/porous backing composite of the present invention can be utilized in an orthopaedic device for implantation in the body of an animal. For example, the crosslinked polymeric/ porous backing composite of the present invention can be utilized in implantable orthopaedic devices, such as knee, hip, shoulder, and elbow prostheses, where the porous backing component is secured to an anchoring bone by any appropriate method, and the polymeric bearing component has a surface upon which a natural bone structure or a prosthetic component articulates.

Embodiments of the invention will now be described by way of example with reference to the accompanying drawings, in which:
FIG. 1 is a perspective view of an implantable modular composite glenoid prosthesis having a polymeric bearing component and a metallic backing component;
FIG. 2 is a schematic illustration of an enlarged cross sectional view of a portion of the backing component of FIG. 1; and
FIG. 3 is schematic illustration similar to FIG. 2 but showing a mass of polymeric material of the bearing component advanced into a portion of the pores of the backing component of FIG. 1.

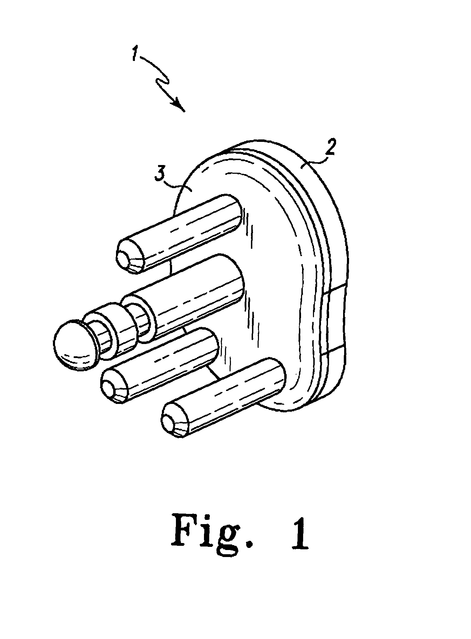

Referring to the drawings, FIG. 1 shows a glenoid prosthesis 1 which includes a polymeric bearing component 2 secured to a rigid, porous, backing component 3. The backing component 3 is a rigid material having greater modulus of elasticity than the bearing component 2 for improved stress transfer to the adjacent bone. The backing component 3 can have a textured portion, can have a porous layer, or can be completely porous to provide sites to anchor the polymeric bearing component 2. In particular, the backing component 3 can be made from any suitable biocompatible rigid material that can be fabricated with a textured portion, with a porosity layer, or fabricated such that the backing component 3 is completely porous.

The backing component 3 can be fabricated from materials such as, for example, porous metal, ceramic, polymeric materials, or a combination of these materials. Examples of metals backing component 3 can be fabricated from include, but are not limited to, titanium alloys and CoCr alloys. Examples of ceramic materials backing component 3 can be fabricated from include, but are not limited to, alumina, zirconia, or blends of these ceramic materials. In addition, backing component 3 can be fabricated from various combinations of the aforementioned materials. For example, backing component 3 can include combinations of metal, ceramic, or polymer materials which have a greater stiffness as compared to the bearing material.

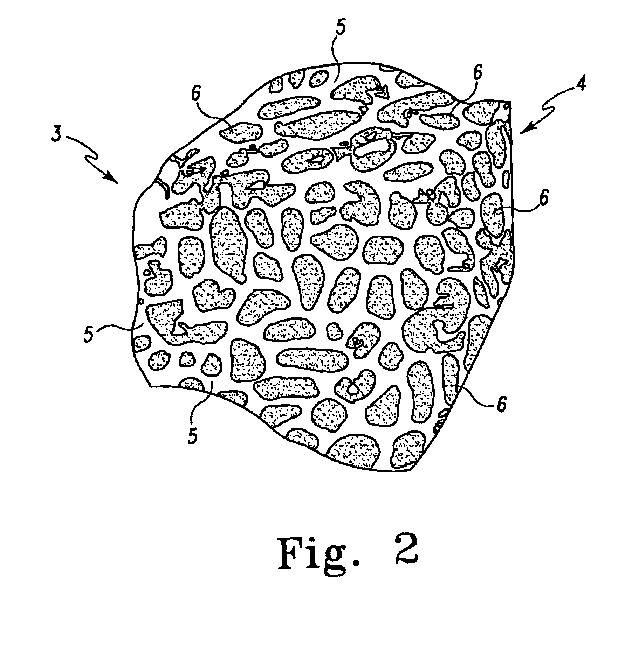

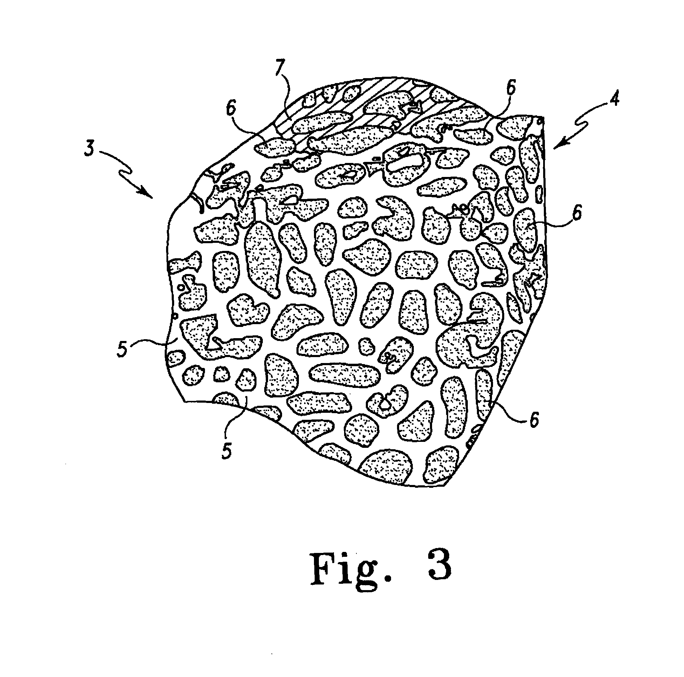

FIG. 2 is an enlarged cross sectional view of a portion of the backing component 3. As shown in FIG. 3, in this particular illustrative embodiment, backing component 3 is fabricated from an open-celled lattice material 4 (e.g. a metallic material). The material 4 includes open spaces or pores 5 interconnected by ligaments 6. The pores 5 of the material 4 can, for example, form a three dimensional network of continuously connected channels that define a bulk volume porosity as previously described. Such a network provides permeability and a high surface area which are desirable conditions for the growth of new bone. In particular, the use of a material having a volume with pores is complementary to the microstructure of natural cancellous bone, and thus will enable that portion of the prosthesis device which is in contact with the natural cancellous bone to incorporate new bone into the prosthesis.

Now turning to polymeric bearing component 2, this component is made from a polymeric material typically having enhanced bearing surface properties as compared to the material of the backing component 3. Polymeric bearing component 2 as used herein includes any work piece of polymeric material that has a bearing surface defined thereon or can have a bearing surface defined thereon with further processing. The term "polymeric" as used herein is intended to include both homopolymers, copolymers, and oriented materials. In particular, bearing component 2 can be made from any medical grade polymeric material which may be implanted into the body of an animal (e.g. the body of a human patient) and be capable of having its viscosity reduced sufficiently with the application of thermal energy such that it will deformation flow into, for example, the pores of the backing component 3. A specific example of such a polymeric material is medical grade polyethylene. The term "polyethylene", as defined herein, includes polyethylene, such as a polyethylene homopolymer, high density polyethylene, high molecular weight polyethylene, high density high molecular weight polyethylene, or any other type of polyethylene utilized in the construction of a prosthetic implant. A more specific example of such a polymer is medical grade ultrahigh molecular weight polyethylene (UHMWPE), such as crosslinked UHMWPE.

The polymeric material bearing component 2 is made from can initially be in an unconsolidated form, such as a powder. What is meant herein by the term "powder" is resin particles sometimes referred to as "flakes". Prior to securing the bearing component 2 to the backing component 3, the flakes of polymeric material are consolidated into a polymeric work piece using any one of a number of well known consolidation techniques. After consolidation, the work piece is subjected to a crosslinking process. Crosslinking the polymeric material after consolidation results in a polymeric bearing component having enhanced mechanical properties as compared to a polymeric bearing component made from polymeric flakes which are crosslinked prior to consolidation.

With respect to crosslinking, while there is no intent to be limited by any particular mechanism, it is believed by persons skilled in the art that irradiation of a polymeric material, such as UHMWPE, initially causes the bonds in the polyethylene chain to be broken by the high energy radiation to form free radicals. Cleavage can occur between carbon and hydrogen atoms in the chain to form a large polyethylene free radical of essentially undiminished molecular weight and a hydrogen free radical. At the other extreme, cleavage can occur between adjacent carbon atoms near the centre of the chain resulting in a broken chain with two shorter polyethylene free radicals. Other reactions may occur such as grafting (when a free radical at the end of one chain reacts with a free radical in the centre of another chain) or the reacting of a short polyethylene chain with a hydrogen free radical to form lower molecular weight molecules. However, most of these free radicals rapidly recombine with other free radicals in the vicinity to produce cross links between chains thereby forming one extremely large molecule of near infinite molecular weight. There is ample evidence for the formation of one extremely large molecule of near infinite molecular weight based on the physical properties of irradiated samples.

One way of crosslinking the consolidated polymeric work piece is to expose it to radiation, such as gamma radiation. Such exposure may be in the exemplary range of about 0.5 Mrads to about 150 Mrads. A specific example of a crosslinked polymeric material that can be utilized in the construction of a device to be implanted in the body of an animal, such as the bearing component 2 described herein, is crosslinked UHMWPE. As alluded to above, a crosslinked UHMWPE work piece can be obtained by first consolidating non-crosslinked UHMWPE flakes into a work piece, and then irradiating the non-crosslinked UHMWPE work piece with gamma radiation. In the alternative, already consolidated work pieces of UHMWPE are commercially available which can be crosslinked via the exposure to radiation. Examples of commercially available non-crosslinked UHMWPE which can be irradiated to obtain crosslinked UHMWPE such as those sold under the trade marks GUR 1050 (having a molecular weight of about 5 million to about 6 million) and GUR 1020 (having a molecular weight of about 3 million to about 4 million) both of which are available from Ticona, located in Summit, New Jersey. In an additional alternative, consolidated and crosslinked work pieces of UHMWPE are also commercially available and can be utilized in the present invention as the material for the bearing component 2.

As indicated above, one manner by which polymeric materials are crosslinked is by gamma irradiation, although other manners such as electron beam or X-ray radiation may also be used. As previously mentioned, the polymeric material may be irradiated with gamma radiation at a dose from about 0.5 Mrads to about 150 Mrads, illustratively from about 3 to about 50 Mrads, and illustratively from about 3 to about 15 Mrads using methods known in the art. The irradiation process may be optionally performed under vacuum or in an inert or substantially oxygen-free atmosphere.

It should be appreciated that not all free radicals rapidly recombine with other free radicals in the vicinity to produce crosslinks between chains. In particular, some free radicals are long lived and may survive for years if not "quenched". These remaining free radicals can have a detrimental effect on the physical/chemical characteristics of the polymeric material if not quenched. Accordingly, a polymeric material, such as UHMWPE, can be subjected to a post-irradiation free radical quenching process. For example, the free radical containing work piece is placed into a vacuum oven which was subsequently brought under vacuum. To quench substantially all the free radicals present in the polymeric work piece, the temperature of the vacuum oven is then raised to above the melting point of the polymeric material for about 24 hours and then brought to room temperature. Accordingly, it should be appreciated that, as contemplated herein, free radical quenching processes include those processes which subject a polymeric material to heat and reduced pressure to quench free radicals. In any event, the polymeric material subjected to a post-irradiation free radical quenching process will be substantially free of free radicals. What is meant herein by "substantially free of free radicals" is that the polymeric material has a de minimis amount of free radicals therein.

An example of a known irradiation and quenching regimen utilized to crosslink UHMWPE is briefly set forth below:
- Bars of UHMWPE are identified with a job number and bar number.
- The bars are placed into bags, and placed into a fixture inside of a bagging chamber at room temperature. The atmosphere in the bagging chamber is removed so that the pressure therein is less than 1 atmosphere. Each bag with a bar of UHMWPE therein is subsequently sealed with a heat sealer. Each bag with the UHMWPE bar contained therein is then removed from the bagging chamber after returning the chamber to atmospheric pressure. Each bag is inspected for integrity, and packaged into a labelled box.
- The boxes are shipped to a facility for irradiation.
- Dosimeters for the recording of the level of radiation are placed on the outside of the boxes and the bars are irradiated.
- After irradiation the bars are debagged and placed inside of a vacuum oven to begin a free radical quenching process.
- The bars are heated and soaked at the elevated temperature for a time period under a pressure less than 1 atmosphere.
- The bars are then cooled and soaked at a lower temperature for a time period under a pressure less than 1 atmosphere. The bars are then cooled prior to removal from the chamber and placed on cooling racks. An outer oxidized layer of each bar is also removed after soaking. All temperature ramping operations are conducted in Argon after a backfilling operation.

After crosslinking and quenching the work piece is shaped or formed into a size and mass suitable for attaching to the backing component 3. For example, the work piece can be machined or otherwise formed into a bearing component which has a complementary perimeter profile to the backing component. In addition, the bearing component can be machined or otherwise formed to enhance the characteristics of the bearing surface defined thereon. While the above discussion describes a sequence of consolidation, crosslinking, quenching, and shaping it should be appreciated that other sequential orders are contemplated. For example, it is contemplated that a consolidated work piece can be shaped and then crosslinked and quenched.

As previously described, to attach the polymeric bearing component 2 to the backing component 3 the polymeric bearing component 2 is heated to supply the thermal energy necessary to sufficiently reduce the viscosity of the polymeric material such that it becomes impregnated into the pores of the backing component with the application of pressure. One way of accomplishing this is to elevate the temperature of the backing component 3 to a level at which sufficient thermal energy is transferred to the polymeric component 2 so as to locally reduce the viscosity of the polymeric material. The lowering of the viscosity of the polymeric material reduces the mechanical properties of the material such that with the application of sufficient force the polymeric material interdigitates with the pores of the backing component 3 as schematically shown in FIG. 3. In particular, FIG. 3 illustrates a mass of polymeric material 7 advanced into the pores 5 of the material 4 of the backing component 3. While FIG. 3 only shows a portion of the pores 5 being filled with polymeric material, it should be appreciated that the method of heating the polymeric material 7 to reduce its viscosity and then applying pressure to force the polymeric material 7 into the pores 5 of the backing component 3 can result in substantially all of the pores 5 of the backing component 3 being filled with the polymeric material 7. Furthermore, while FIG. 3 shows an open porous structure, it should be understood that a mass of polymeric material can also interdigitate with the pores of a backing component that has a closed porous structure so as to result in a mechanical bond of sufficient strength to hold the polymeric bearing component secure to the rigid backing component.

The heating of the polymeric component 2 can be controlled so that only a narrow zone of the polymeric material adjacent to, and making contact with, the surface of the backing component 3 is lowered in viscosity. The low thermal conductivity of the polymeric material and the limited amount of thermal energy imparted to the backing component 3 restricts the amount of material for which the viscosity will be lowered. The outside surface material of the bearing component which articulates against a mating orthopaedic device or a bone structure is totally unaffected by the bonding process, as very little thermal energy is transmitted to that surface. Thermal energy input should also be controlled to avoid heating the polymeric material to excessive temperatures, since the exposure of a polymeric material to such temperatures can cause the breaking of the molecular chain and the reduction of the molecular weight of the material. In the case of UHMWPE, as the molecular weight decreases the resistance to wear also decreases.

An example of a procedure which can be utilized to fabricate an orthopaedic device of the present invention is set forth below:
- Machine a consolidated, crosslinked, quenched, UHMWPE work piece into an appropriate bearing component having a desired perimeter shape.
- Position a porous metallic backing component having a perimeter profile that is complementary to the perimeter profile of the above described UHMWPE bearing component at the base of a mould cavity of a compression moulding apparatus.
- Position the UHMWPE bearing component atop the metallic backing component and mould using appropriate cycle parameters for temperature (e.g. about 135°C to about 260°C), pressure (e.g. about 1000 psi to about 70,000 psi), and time so as to ensure sufficient interface shear strength between the UHMWPE bearing component and the metallic backing component. Note that in light of the discussion set forth herein, the cycle parameters utilized to obtain a backing component/bearing component composite having a desired interface shear strength will be determined for each particular application. Note that either a one-soak moulding cycle (melt soak only) or a two-soak moulding cycle (melt soak and recrystallisation soak) can be utilized.
- Sterilize the metallic backing component/UHMWPE polymeric bearing component composite using a non irradiation method to avoid introducing free radicals into the UHMWPE polymeric bearing component (e.g. gas plasma).

## Claims

1. A method of preparing an implantable orthopaedic device that includes (i) a consolidated bearing component made from a crosslinked polymeric material and (ii) a porous backing component attached to the bearing component, wherein the backing component has a greater modulus of elasticity than the bearing component, the method comprising advancing a mass of the polymeric material of the consolidated bearing component into the pores of the backing component to cause the backing component to be mechanically attached to the bearing component.

2. The method of claim 1, which includes subjecting the consolidated bearing component to a free radical quenching process.

3. The method of claim 1, which includes heating the bearing component to supply thermal energy necessary to reduce the viscosity of the polymeric material prior to advancing the mass of the polymeric material of the consolidated bearing component into the pores of the backing component.

4. The method of claim 1, in which the crosslinked polymeric material of the bearing component includes crosslinked UHMWPE.

5. The method of claim 1, in which the backing component has an open porous structure.

6. The method of claim 1, in which the pores of the backing component define a bulk volume porosity from about 25% to about 75%.

7. The method of claim 1, in which the backing component is made from a metallic material.

8. The method of claim 1, which includes subjecting the consolidated bearing component to a sterilization process.

9. A method of preparing an implantable orthopaedic device, the method comprising:
consolidating flakes of polymeric material into a polymeric work piece;
subjecting the polymeric work piece to a process to cause crosslinking of the polymeric material of the polymeric work piece; and
advancing a mass of the polymeric material of the polymeric work piece into pores of a backing component to cause the backing component to be mechanically attached to the polymeric work piece.

10. A method of preparing an implantable orthopaedic device that includes (i) a polymeric component made from a polymeric material and (ii) a backing component attached to the polymeric component, wherein the backing component has (A) pores defined therein and (B) a greater modulus of elasticity than the polymeric component, the method comprising:
consolidating flakes of a polymeric material into the polymeric component;
subjecting the polymeric component to a process to cause crosslinking of the polymeric material of the polymeric component;
subjecting the polymeric component to a process to cause quenching of substantially all free radicals present in the polymeric component; and
advancing a mass of the polymeric material of the polymeric component into pores of the backing component to cause the backing component to be attached to the polymeric component.
